# EUROPEAN PATENT APPLICATION

(11) **EP 2 700 385 A1**
(43) Date of publication of application: **26.02.2014**
(21) Application number: 11863889.9
(22) Date of filing: 13.09.2011
(51) Int. Cl.: A61F 5/08

(54) **NOSE TENSIONER AND OPENER AND NOSE TIP LIFTER FOR THE TREATMENT OF OBSTRUCTIVE SLEEP APNOEA, SNORING AND RESPIRATORY OBSTRUCTION**

(30) Priority: 18.04.2011 BR PI1105463
(71) Applicant: Godoy Roberto Ferreira, Paulo, São Paulo - SP 01327-002 (BR); Godoy Carpovinski Ouriques, Erica, São Paulo - SP 01327-002 (BR)
(72) Inventor: Godoy Roberto Ferreira, Paulo, São Paulo - SP 01327-002 (BR); Godoy Carpovinski Ouriques, Erica, São Paulo - SP 01327-002 (BR)
(74) Representative: Lorente Berges, Ana
(86) International application number: PCT/BR2011/000322
(87) International publication number: WO 2012/142683

(57) **Abstract**

(EN)The invention relates to a device for external use on the nose with a special and innovative action mechanism, with hooks that are inserted into the nostrils and apply a vigorous mechanical action, tensioning the (nostril dilator) nose muscle, the (nostril opening) nose wing and upper lip elevator muscles, and the nose wing greater cartilage (greater alar cartilage), this tension applied to the three structures widely opening the nostrils, tensioning and lifting the nose tip and cancelling the action of the nasal septum depressor muscle. The correct position is maintained by pulling the upper end of the device vertically, towards the root of the nose, and attaching it to the ridge of the nose with adhesive tape, thus stopping obstructive sleep apnea, snoring, nose, soft palate and uvula obstructions. The apparatus is formed in a single rigid piece (figure 1) weighing less than 10 gr., made of titanium, plastics, aluminum and paper, inter alia, without any joints, with two vertical reeds (2) that are 2 cm long and 0.5 cm wide, with hooks at the lower tips (3) that measure 1.0x0.5 cm and are inserted into the nostrils, the reeds being joined at the upper end (1), which measures 1.0x1.0 cm, giving the device an inverted Y shape.

## Description

This patent of invention concerns a very light, rigid device, less than 10 grams, made in plastic, aluminum, titanium, paper and others, for external use, on the nose, which tensions the nasalis muscles (nostril dilator) and opens the greater alar cartilage and, consequently, opens the nostrils wide, lifting the nose tip and repositioning all internal and external structures of the nose, making a great amount of air enter through the nose only and not through the mouth anymore, bringing breathing back to normal and stopping snoring and obstructive sleep apnea, allowing for a deep and restorative sleep, being effective for nose congestion, nasal septum deviation, turbinate and adenoid hypertrophy, rhinitis, sinusitis and other obstructive respiratory disorders, where air passage is blocked, as in nasal stenosis and obstructions at the level of the nasopharynx, soft palate and uvula. The device is built with two vertical reeds that are 2 cm long and 0.5 cm wide, the lower tips of which being 1.0 x 0.5 cm hooks that are introduced into the nostrils, the reeds are joined together in the upper end that is 1.0 x 1.0 cm, providing the device with an inverted "Y" shape, the hooks that are introduced into the nostrils tension the muscles and the nose cartilage and vigorously open the nostrils, lifting the nose tip and canceling the action of the nasal septum depressor muscle and separate tissues that previously blocked the nose, stopping snoring, obstructive sleep apnea and respiratory obstructions. The tension exerted by the lower reeds is maintained by the upper tip, which is attached to the nasal dorsum, which is bony and firm, located 2 cm from the nasal fossae. Its action is mechanical, it may be disposable or of continued use.

Some filed patents for treating snoring, obstructive sleep apnea and upper airway obstructions are already known. These are movable orthodontic devices, similar to those used for the esthetic correction of teeth, comprising two parts, which are fitted to the upper and lower arch teeth, making the mandible move in relation to the maxilla, pulling the mandible forward, causing the lower teeth to advance farther than the upper ones, which is called prognathism, and pulling the tongue forward. There is an orifice between two plates, so that the person can breathe through the mouth and sleep with mouth open in order to be able to breathe. There are also the so-called flexible nasal strips, with two parallel plastic bars in disposable adhesive paper, which are horizontally attached to the nose skin 2.0 cm away from the nostrils, its action occurs when the attached plastic attempts to return to its original position and slightly pull the lateral skin of the nose, theoretically to improve the nasal orifice by pulling the wing of the nose outwards.

The action mechanism of these inventions is totally different from the invention presented here, in the orthodontic braces example, it is used inside the mouth, its purpose is to allow the person to breathe through the mouth, which causes nasal breathing to worsen and gradually lose its function; in addition, the temporomandibular joint is forced forward, leading to an injury to the meniscus and articular disc of the TMJs (temporomandibular joints), without improving physiological breathing, as we should breathe 100% through the nose and should not breathe through the mouth at all.

Regarding the so-called flexible nasal strips, these do not show any similarity to the construction and action mechanism of the invention presented here and serves only as a reminder, as they are reportedly indicated for respiratory disorders. Their action is limited, they are not introduced into the nostril; rather, they are horizontally attached to the middle of the nose, away from the nostrils and the nose tip, and do not open the nostrils, as they only slightly pull the nose skin outwards and easily detach from the skin; this is the action mechanism of this flexible strip, while the invention presented here vigorously tensions the muscles and the nasal cartilage, which are strong and heavy structures, by its lower tips as a hook, which enter the nostrils, pull and vertically open the nostrils, lifting the nose tip towards the root of the nose (nasal pyramid), where the adhesive tape is attached.

In view of these problems and the non-existence so far of an ideal rigid device, with physical characteristics and vigorous action as the one presented here, for use on the nose to correct snoring and obstructive sleep apnea and other obstructive respiratory disorders, with the purpose to overcome this shortage, the present invention was developed, whose technique described in the invention presented here has special and innovative characteristics both in its shape, design and mechanism of vigorously tensioning the nasal dilator muscles and the wing of nose and upper lip elevator muscles and on the greater alar cartilage and it is attached to the nasal pyramid.

The present invention is intended for the embodiment of a product as a non-flexible, strong and very light, rigid device that has these two action mechanisms: opening the nostrils wide in all directions and lifting the nose tip, suitably correcting the internal and external structures of the nose by pulling the flaccid internal structures so as to prevent them from drooping in the air path during sleep. Thus, it will prevent these tissues from vibrating (cause of snoring) and avoid obstructive sleep apnea and other obstructions of the upper airways. With this invention, a great amount of air easily enters without attrition, restoring the role of the nose of being the only organ that is able to receive, filtrate, warm, moisten and carry air immediately to the lungs and remain open for the passage of the air expelled from the lungs, maintaining good oxygenation and quality of sleep.

The present invention, whose technique is described and presented here, is intended for a product in the form of a device comprising a single piece, very light, non-flexible, rigid, made of titanium, plastic, aluminum, paper, and other materials. Its use is external, a non-pharmacological method of mechanical opening, of universal size, it adapts to all nasal sizes and comprises two vertical reeds that are 2 cm long and 0.5 cm wide, the lower tips of which are 1.0 x 0.5cm hooks that are introduced into the nostrils, the reeds are joined together at the upper end, which is 1.0 x 1.0 cm, providing the device with an inverted "Y" shape. The hooks tension the nasalis muscle (nostril dilator) and the elevator muscle of the wing of the nose and upper lip and also tension the greater alas cartilage and, thus, opens the nostrils wide and lifts the nose tip, canceling the action of the nasal septum depressor muscle, which with aging makes the tip of the nose droop and the nostril is gradually closed, separating the tissues that previously blocked the nose, getting rid of snoring, obstructive sleep apnea and respiratory obstructions. The tension exerted by the lower reeds is maintained by the upper tip, which is vertically pulled upwards and secured with adhesive tape on the skin of the nasal dorsum, which is bony and firm.

To complement the present description, for a better understanding of the characteristics of the present disclosure and according to the preferred embodiment thereof, and as we already built a prototype, the description is accompanied with a set of drawings of the invention, given as a non-limitative example, for a proper understanding of the invention, and these are illustrated as follows:
Figure 1 is a front view of the nasal device, according to its preferred embodiment
Figure 2 is an oblique view
Figure 3 is a side view while in use

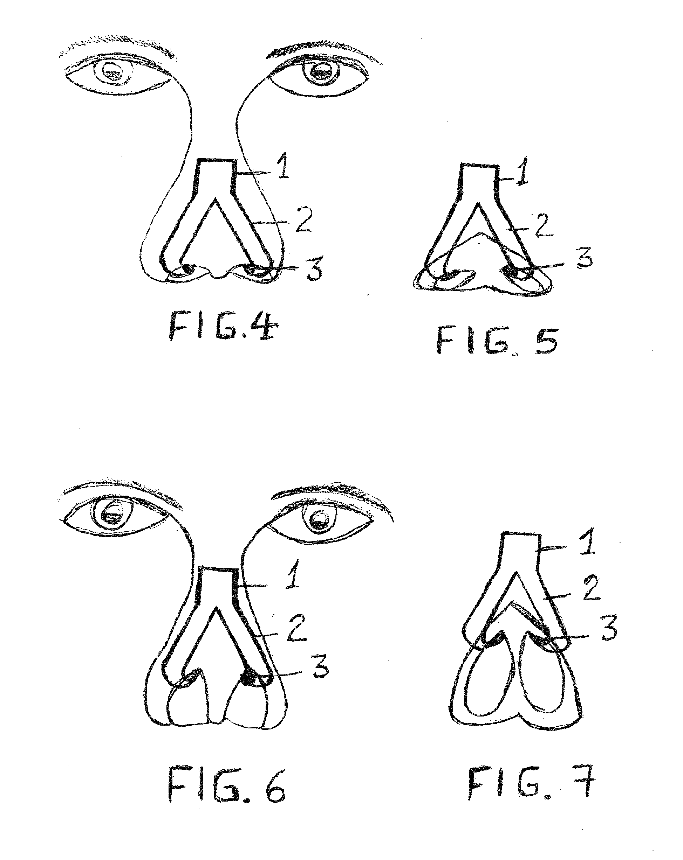

Figure 4 is a front view placed without tensioning
Figure 5 is a front view in detail without tensioning.
Figure 6 is a front view while opening the nostrils and lifting the nose tip, correctly secured.
Figure 7 is a front view in detail while in action

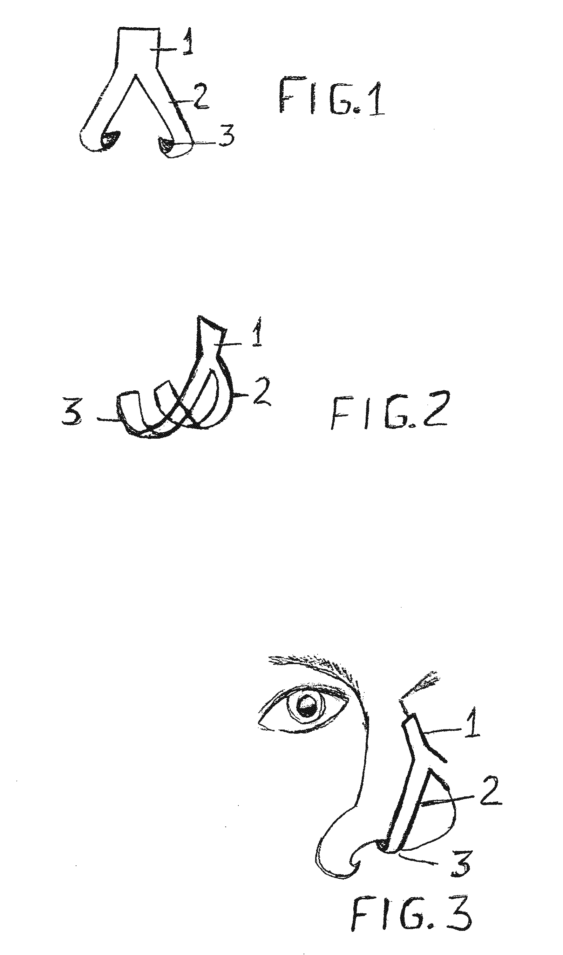

Regarding the illustrated drawings, the present invention is related to a tensioner device and nose opener and nose tip lifter for treating snoring, obstructive sleep apnea and respiratory obstructions, more precisely, it concerns a device comprising two vertical reeds (2) that are 2 cm long and 0.5 cm wide, whose lower tips are 1.0 x 0.5 cm hooks that are introduced into the nostrils, the reeds are joined together at the upper end (I) that is 1.0 x 1.0 cm, providing the device with an inverted "Y" shape, the hooks (3) entering the nostrils, tensioning the nasalis muscle (nostril dilator), the elevator muscle of the wing of the nose and upper lip and tensions and opens the greater alas cartilage and, thus, opens the nostrils wide and lifts the nose tip, canceling the action of the nasal septum depressor muscle (with aging this muscle makes the nose tip droop and the nostril is gradually closed), separating the tissues that previously blocked the nose, getting rid of snoring and obstructive sleep apnea, being effective for nasal congestion, turbinate and adenoid hypertrophy, rhinitis, sinusitis, septal deviation, nasal stenosis and obstructions at the level of the nasopharynx, soft palate and uvula. The mechanical opening prevents waking up during sleep, insomnia and daytime sleepiness. This exerted tension is maintained by the upper end (1), which is 1.0 x 1.0 cm and is attached to the central portion of the nasal dorsum, which is bony and firm, and serves to keep the device at a correct position and support the entire open nose. It is built in a seamless single piece (Fig. 1), less than 10 grams, manufactured in plastic, titanium, aluminum, paper, and other materials.

## Claims

1. "NOSE TENSIONER AND OPENER AND NOSE TIP LIFTER FOR THE TREATMENT OF OBSTRUCTIVE SLEEP APNOEA, SNORING AND RESPIRATORY OBSTRUCTIONS" comprising a device built in a seamless single piece (Fig. 1), **characterized by** in that its special and innovative action mechanism occurs by the vigorous mechanical action of hooks introduced into the nostrils, which tension the nasalis muscle (nostril dilator), the elevator muscles of the wing of the nose and upper lip (nostril opener) and the greater alas cartilage, this tension on the three structures open the nostrils wide, pulls and lifts the tip of the nose, this correct position is maintained by pulling the upper end of the device vertically towards the root of the nose and securing it to the nasal dorsum with an adhesive tape, keeping the nose open and correctly positioned by the device, which is built as a rigid, seamless single piece (Fig. 1), very light, at less than 10 grams, made of titanium, plastic, aluminum, paper, and other materials, comprising two vertical reeds (2), 2 cm in length and 0.5 cm in width, whose lower tips are 1.0 x 0.5 cm hooks (3), which enter the nostrils, the reeds are joined together at the upper end (1) that is 1.0 x 1.0 cm, providing the device with an inverted "Y" shape.
